# EUROPEAN PATENT SPECIFICATION

(11) **EP 2 634 262 B1**
(45) Date of publication and mention of the grant of the patent: **31.05.2017**
(21) Application number: 11836659.0
(22) Date of filing: 28.10.2011
(51) Int. Cl.: G01N 33/50, G01N 33/15, C12N 15/12, C12N 15/11, G01N 33/68, C12Q 1/68

(54) **KIT FOR SCREENING FOR SKIN-ACTIVATING SUBSTANCES AND COMPRISING THE KLOTHO GENE, AND METHOD FOR SCREENING FOR SKIN-ACTIVATING SUBSTANCES USING SAME**
KIT ZUM SCREENING AUF HAUTAKTIVIERENDE SUBSTANZEN MIT DEM KLOTHO-GEN UND VERFAHREN ZUM SCREENING AUF HAUTAKTIVIERENDE SUBSTANZEN DAMIT
TROUSSE DE CRIBLAGE DE SUBSTANCES D'ACTIVATION DE LA PEAU ET COMPRENANT LE GÈNE KLOTHO, ET PROCÉDÉ DE CRIBLAGE DE SUBSTANCES D'ACTIVATION DE LA PEAU À L'AIDE DE CELLE-CI

(30) Priority: 20.10.2011 KR 20110107409; 29.10.2010 KR 20100107000
(43) Date of publication of application: 04.09.2013
(73) Proprietor: Amorepacific Corporation, Seoul 140-777 (KR)
(72) Inventor: CHOI, Hyang Tae, Yongin-si Gyeonggi-do 446-904 (KR); SON, Eui Dong, Yongin-si Gyeonggi-do 446-904 (KR); LEE, Jin Young, Yongin-si Gyeonggi-do 446-904 (KR); NA, Yong Joo, Yongin-si Gyeonggi-do 446-904 (KR); BAE, Ji Hyun, Yongin-si Gyeonggi-do 446-904 (KR)
(74) Representative: Ilgart, Jean-Christophe
(86) International application number: PCT/KR2011/008132
(87) International publication number: WO 2012/057567

(56) References cited:
- EP-A1- 2 209 006
- WO-A1-2008/143145
- WO-A2-01/20031
- FR-A1- 2 908 654
- FR-A1- 2 924 613
- FR-A1- 2 924 947
- JP-A- 2008 232 693
- KR-A- 20080 018 740
- KR-B1- 100 840 144
- KURO-O, M. ET AL.: 'Mutation of the mouse Klotho gene leads to a syndrome resembling ageing' NATURE vol. 390, 06 November 1997, pages 45 - 51, XP002241394
- KUROSU, H. ET AL.: 'Suppression of aging in mice by the hormone Klotho' SCIENCE vol. 309, 16 September 2005, pages 1829 - 1833, XP055047765
- LI, S-A. ET AL.: 'Immunohistochemical localization of Klotho protein in brain, kidney, and reproductive organs of mice' CELL STRUCTURE AND FUNCTION vol. 29, 2004, pages 91 - 99, XP055100902
- OHNISH, M. ET AL.: 'Dietary and genetic evidence for phosphate toxicity accelerating mammalian aging' FASEB JOURNAL vol. 24, September 2010, pages 3562 - 3571, XP055100907
- NAKATANI, T. ET AL.: 'In vivo genetic evidence for klotho-dependent, fibro blast growth factor 23(Fgf23)-mediated regulation of systemic phosphate home ostasis' FASEB JOURNAL vol. 23, February 2009, pages 433 - 441, XP055100909
- H. KUROSU: "Suppression of Aging in Mice by the Hormone Klotho", SCIENCE, vol. 309, no. 5742, 16 September 2005 (2005-09-16), pages 1829-1833, XP055047765, ISSN: 0036-8075, DOI: 10.1126/science.1112766

## Description

### Technical Field

The present invention relates to the use of kit for screening moiturizing substances and a method for screening moisturizing substances using the same, in which moisturizing substances are screened by isolating and purifying substances, which induce the expression of Klotho gene in skin cells, using the kit comprising Klotho gene known as moisturizing gene.

### Background Art

Skin aging naturally occurs with time or is mainly caused by sun exposure. As the skin ages, the time for the horny layer of the epidermis to be replaced is increased so that the horny layer becomes thicker, and the content of elastic components and moisturizing components in the dermis decreases or the arrangement thereof changes so that the elasticity and water content of the dermis decrease, resulting in wrinkle formation. Photoaging caused by UV radiation is a cause of skin aging, and methods capable of preventing the change in the skin caused by UV radiation have been actively studied. Further, methods for preventing age-related aging, that is, genetically programmed aging, have also been studied. Such efforts have been focused on finding mechanisms that control aging.

Such efforts include studies on the Klotho gene. Klotho- knockout mice show arteriosclerosis, vascular calcification, soft tissue calcification, emphysema, activity reduction, gonadal dysgenesis, sterility, skin atrophy, ataxia, hypoglycemia and hyperphosphatemia, which are related to an increase in the concentration of 1,25(OH)₂D₃ (Mutation of the mouse klotho gene leads to a syndrome resembling ageing, Nature 1997 ; 390.45-51). On the other hand, an increase in the expression of the Klotho protein indicates increases in life expectancy, insulin resistance and IGF-1 resistance (Kurosu et al., 2005). It was reported that single nucleotide polymorphisms in the human Klotho protein are associated with a reduction in life expectancy, osteoporosis, stroke and coronary artery disease (Arking et al., 2002. Kawano et al., 2002; Mull in et al., 2005. Ogata et al., 2002; Yamada et al., 2005).

Previous studies show that Klotho is expressed mainly in human kidneys and is also expressed in the placenta, the prostate and the small intestines. The recognition of importance of Klotho has increased, but the expression of Klotho in skin cells, including normal human keratinocytes, fibroblasts or melanocytes, has not yet been reported, and a study on a substance capable of increasing the expression of Klotho has also been reported.

### Disclosure

### Technical Problem

Accordingly, the present inventors have constructed a kit capable of increasing the expression of Klotho protein in skin cells and have identified the expression of Klotho protein in skin cells, including keratinocytes, fibroblasts and melanocytes, using the constructed kit, suggesting that moisturizing substances, can be screened using the kit, thereby completing the present invention.

Therefore, it is an object of the present invention to provide a kit for screening moisturizing substances by determining whether the Klotho gene is expressed in skin cells, and a method for screening moisturizing substances using the kit.

### Technical Solution

In order to accomplish the above object, the present invention provides a kit for screening moisturizing substances by screening substances which increases the expression of Klotho gene in skin cells, the kit comprising Klotho gene. Klotho gene amplification primers of SEQ ID NOS: 1 and 2, a probe of SEQ ID NO: 3, and skin cells.

The present invention also provides a method for screening a moisturizing substance using the kit.

### Advantageous Effects

The inventive kit for screening skin-activating substances can quickly and conveniently screen moisturizing substances, by determining whether Klotho gene is expressed in skin cells.

### Description of Drawings

FIG. 1a shows the results of RT-PCR amplification performed to determine whether Klotho gene is expressed in human embryonic kidney cells (HEK293), keratinocytes (NHK) and fibroblasts (NHF), and FIG. 1b shows the results of RT-PCR amplification performed after identifying the expression of Klotho in human melanocytes. In FIGS. 1a and 1b, NHM-promoblack and NHM-DP indicate melanocytes derived from dark-skinned persons, and NHM-MP indicates melanocytes derived from Asian.
FIG. 2 is a graphic diagram showing the results of quantitative real-time PCR performed to determine the expression levels of Klotho gene in human embryonic kidney cells (HEK293), keratinocytes (NHK) and fibroblasts (NHF).
FIG. 3 is a graphic diagram showing the results of quantitative real-time PCR performed to determine the expression level of Klotho in keratinocytes at 2 days and 8 days after treatment with retinoic acid (RA) and retinol (ROL).
FIG. 4 is a graphic diagram showing a decrease in the expression level of Klotho gene in Klotho-knockdown human neonatal epidermal keratinocytes (HEK).
FIG. 5 shows the change in the expression level of MMP-9 in Klotho-knockout human fetal epidermal keratinocytes (HEK).
FIG. 6 is a graphic diagram showing the change in MMP-9 activity in human neonatal epidermal keratinocytes (HEK) treated with a recombinant Klotho protein.
FIG. 7 is a graphic diagram showing the changes in the mRNA expression level of HAS2 in human neonatal epidermal keratinocytes (HEK) at 2 days and 8 days after treatment with 1 *µ*g/ml and 0.5 *µ*g/ml of a recombinant Klotho protein.
FIG. 8 is a graphic diagram showing the change in the expression level of p21 in Klotho-knockdown human neonatal dermal fibroblasts (HDF).

### Best Mode

The present invention relates to a method capable of identifying the expression of Klotho protein in skin cells. According to the method of the present invention, skin-activating substances capable of inducing the expression of the anti-aging gene Klotho in skin cells can be screened. Examples of the skin-activating substances include anti-aging substances, which reduce skin wrinkles or improve skin elasticity or make the skin soft, whitening substances, which brighten skin tone or reduce skin pigmentations, such as discolorations, freckles, blemish, and skin tanning by sunlight, and skin-moisturizing substances.

The inventive screening kit capable of identifying the expression of Klotho gene in skin cells comprises Klotho gene, Klotho gene amplification primers of SEQ ID NOS: 1 and 2, a probe of SEQ ID NO: 3 (see Table 1), and skin cells, and can identify moisturizing substances by screening substances which increase the expression level of Klotho gene in the skin cells.

**Table 1**

| SEE ID NO: | Name | Sequence |
|---|---|---|
| 1 | Forward primer | 5'-TGGAAACCTTAAAAGCCATCAAGC-3' |
| 2 | Reverse primer | 5'-CCACGCCTGATGCTGTAACC-3' |
| 3 | TaqMan™ fluorogenic probe | 5'-FAM- CTGTGCCACTCGAAACCATCCATGAGGG-BHQ- 1-3' |

In one embodiment of the present invention, the skin cells may be normal human keratinocytes (NHK), normal human fibroblasts (NHF) or normal human melanocytes (NHM), but are not limited thereto.

The present invention also relates to a method for screening moisturizing substances using the above kit, the method comprising the steps of: treating skin cells with candidates and determining whether Klotho gene is expressed in the skin cells treated with the candidates; and isolating and purifying substances, which increase the expression of the Klotho gene, among the candidates.

In one Example of the present description, it was found using the above kit that retinoic acid and retinol, known as anti-aging substances, increased the expression of Klotho gene, suggesting that the screening method according to the present invention is suitable for screening anti-aging substances and skin-activating substances, which have not yet been known.

Hereinafter, the present invention will be described in further detail with reference examples and test examples. It is to be understood, however, that these examples are for illustrative purposes only and are not intended to limit the scope of the present invention, and various modifications, additions and substitutions are possible, without departing from the scope and spirit of the invention as disclosed in the accompanying claims.

### Mode for Invention

### Test Example 1: Expression of Klotho in skin cells

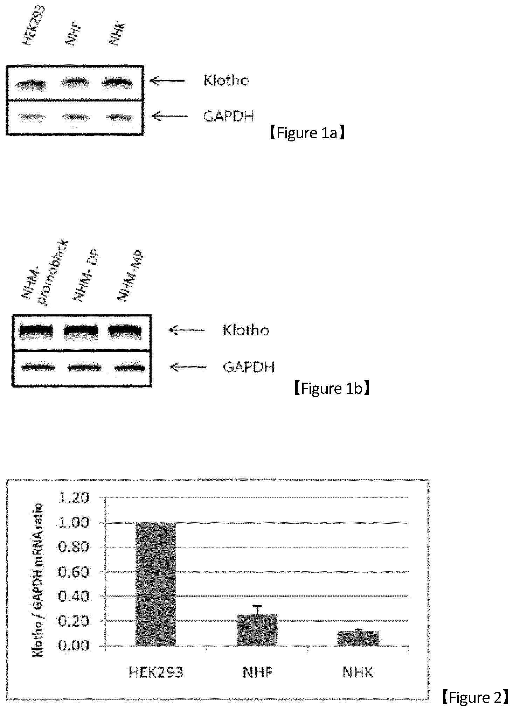

In order to examine whether Klotho protein is expressed in skin cells, primers of SEQ ID NOS: 1 and 2, which can amplify Klotho gene (NCBI accession No. NM-004795), were constructed. Then, RT-PCR was performed using the primer set and a probe of SEQ ID NO: 3 (TaqMan™ fluorogenic probe) in order to detect Klotho mRNA in the cDNA of keratinocytes (NHK), fibroblasts (NHF), NHM-promoblack (Normal Human Epidermal Melanocytes (NHEM) juvenile foreskin, Promocell : melanocytes derived from dark-skinned persons), NHM-DP (NHM-dark pigment, Invitrogen : melanocytes derived from dark-skinned persons) and NHM-MP (NHM-moderate pigment, Invitrogen : melanocytes derived from Asian). The PCR reaction was performed for 35 cycles, each consisting of denaturation at 94°C for 30 sec, annealing at 55°C for 30 sec, and extension at 72°C for 60 sec. As a positive control, the cDNA of human embryonic kidney cells (HEK293) known to express Klotho gene was used. The results of the RT-PCR amplification are shown in FIGS. 1a and 1b. As can be seen from the results in FIGS. 1a and 1b, Klotho gene was expressed in all the keratinocytes, fibroblasts and melanocytes that were used in the present invention.

In addition, the expression level of Klotho mRNA in each type of cells was analyzed by quantitative real-time PCR, and the results of the analysis are shown in FIG. 2.

### Test Example 2: Examination of expression levels of Klotho mRNA in keratinocytes treated with retinoic acid (RA) and retinol (ROL)

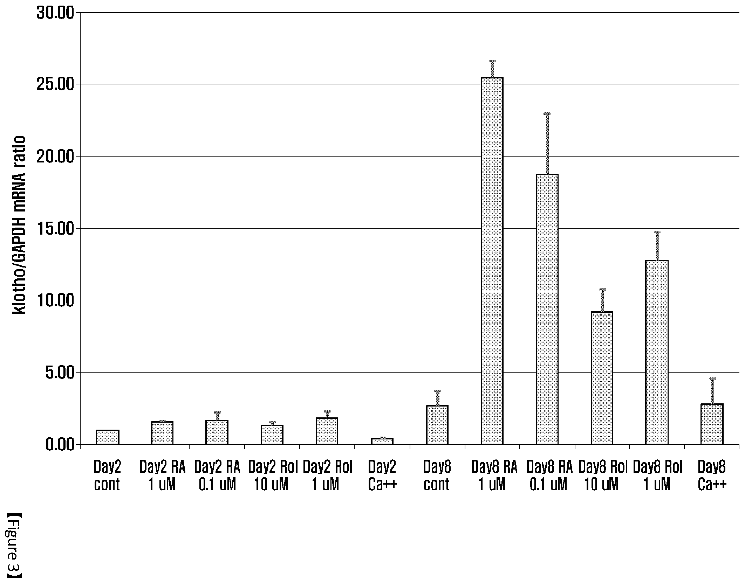

In order to examine the effects of retinoic acid and retinol (known as anti-aging substances) on the expression of Klotho in keratinocytes, quantitative real time PCR was performed under the same conditions as described in Test Example 1, and the expression levels of Klotho protein were examined at 2 days and 8 days after treatment of the cells with each of 0.1 µM and 1 µM of retinoic acid and 1 µM and 10 µM of retinol. The results of the examination are shown in FIG. 3.

As can be seen from the results in FIG. 3, the expression level of Klotho protein significantly increased at 8 days after treatment with each of retinoic acid and retinol.

### Test Example 3: Knockdown of Klotho gene in human neonatal epidermal keratinocytes (HEK)

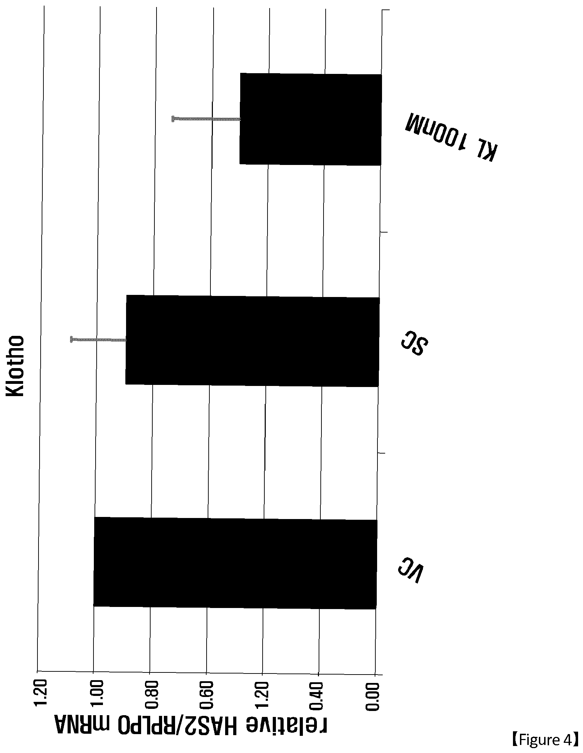

Human neonatal epidermal keratinocytes (HEK; Lonza, NHEK-Neo-Neonatal Normal Human Epidermal Keratinocytes, Pooled) were dispensed in medium (KBM-gold, Lonza) in a 6-well plate at a density of 1x10⁵ cells per well and cultured at 37°C for 24 hours, after which the medium was replaced with OPTI-MEM medium (GIBCO). Meanwhile, human neonatal epidermal keratinocytes were cultured in OPTI-MEM medium (GIBCO) containing 100 nM of siRNA (small interfering RNA; Dharmacon, ON-TARGET*plus*) having a sequence complementary to Klotho gene and 10 µL of transfection reagent (RNAiMAX, Invitrogen) at 37°C for 20 minutes, after which these cells were added to the above cultured cells and incubated at 37°C for 6 hours. Then, the cells were incubated in DMEM medium at 37°C for 4 days. As control groups, HEK cells treated with siRNA (sc in FIG. 4) and HEK cells treated with only transfection reagent (RNAiMAX) without siRNA (VC in FIG. 4) were used.

To examine whether the Klotho gene was knockdown, quantitative real-time PCR was performed under the same conditions as described in Test Example 1, and the results of the PCR are shown in FIG. 4.

As can be seen from the results in FIG. 4, the expression of Klotho gene in the cells treated with siRNA and transfection reagent significantly decreased.

### Test Example 4: Increase in expression level of MMP-9 in Klotho-knockdown human neonatal epidermal keratinocytes (HEK)

In order examine the influence of Klotho protein on MMP-9 known to degrade collagen to cause skin aging, the change in the expression level of MMP-9 in Klotho-knockdown human neonatal epidermal keratinocytes (HEK) was analyzed by RT-PCR.

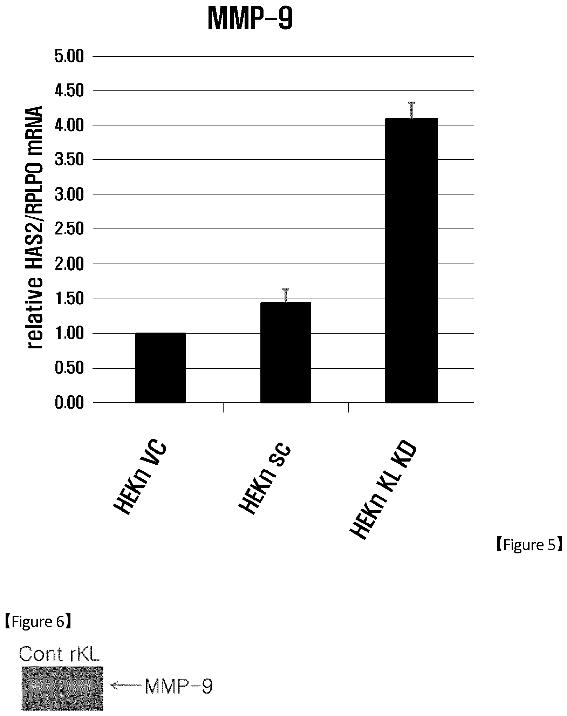

Human neonatal epidermal keratinocytes (HEK; Lonza, NHEK-Neo-Neonatal Normal Human Epidermal Keratinocytes, Pooled) were dispensed in medium (KBM-gold, Lonza) in a 6-well plate at a density of 1x10⁵ cells per well and cultured at 37 °C for 24 hours, after which the medium was replaced with OPTI-MEM medium (GIBCO). Meanwhile, human neonatal epidermal keratinocytes were cultured in OPTI-MEM medium (GIBCO) containing 100 nM of siRNA (small interfering RNA; Dharmacon, ON-TARGET*plus*) having a sequence complementary to Klotho gene and 10 µL of transfection reagent (RNAiMAX, Invitrogen) at 37°C for 20 minutes, after which these cells were added to the above cultured cells and incubated at 37°C for 6 hours. Then, the cells were incubated in DMEM medium at 37°C for 4 days. As control groups, HEK cells treated with siRNA (HEKn sc in FIG. 5) and HEK cells treated with only transfection reagent (RNAiMAX) without siRNA (HEKn VC in FIG. 5) were used.

RNA was extracted from the cultured HEK cells using a RNeasy mini kit (Qiagen) and subjected to RT-PCR using a Superscript III kit (Invitrogen) to synthesize cDNA. The synthesized cDNA was subjected to quantitative real-time PCR using a probe (TaqMan® Gene Expression Assays) for MMP-9, thereby analyzing the expression pattern of MMP-9 mRNA. The results of the analysis are shown in FIG. 5. As can be seen from the results in FIG. 5, the expression level of MMP-9 in the Klotho-knockdown HEK cells increased, suggesting that MMP-9 activity in the Klotho-knockdown cells increases to stimulate skin aging.

### Test Example 5: Decrease in activity of MMP-9 in human neonatal epidermal keratinocytes treated with recombinant Klotho protein

Human neonatal epidermal keratinocytes (HEK; Lonza, NHEK-Neo-Neonatal Normal Human Epidermal Keratinocytes, Pooled) were dispensed in medium (KBM-gold) in a 6-well plate at a density of 5x10⁴ cells per well and cultured at 37°C for 24 hours, after which the cells were treated with 1 µg/ml of a recombinant Klotho protein (R&D systems, Recombinant Human Klotho) and incubated for 8 days. As a control group, HEK cells not treated with Klotho protein were used.

The HEK culture was loaded on gelatin gel and analyzed by zymography, and the results of the analysis are shown in FIG. 6.

As can be seen from the results in FIG. 6, the activity of MMP-9 in the HEK cells treated with the recombinant Klotho protein was lower than that in the control group. Because the MMP-activity in the cells was decreased by treatment with Klotho protein, gelatin degradation in the cells decreased, suggesting that Klotho protein has the effect of maintaining the basal membrane.

### Test Example 6: Increase in expression of HAS2 mRNA in human neonatal epidermal keratinocytes (HEK) treated with recombinant Klotho protein

In order to examine the influence of Klotho protein on the expression of hyaluronic acid synthase 2 (HAS2) known as a skin-moisturizing factor, human neonatal epidermal keratinocytes (Lonza, NHEK-Neo-Neonatal Normal Human Epidermal Keratinocytes, Pooled) were treated with a recombinant Klotho protein, and the expression pattern of HAS mRNA in the cells was analyzed.

Human neonatal epidermal keratinocytes (HEK) (Lonza, NHEK-Neo-Neonatal Normal Human Epidermal Keratinocytes, Pooled) were dispersed in medium (KBM-gold) in a 6-well plate at densities of 2x10⁵ cells per each well of 3 wells and 5x10⁴ cells per each well of 3 wells and cultured at 37°C for 24 hours, after which the cells were treated with a recombinant Klotho protein at concentrations of 1 and 0.5 µg/ml as shown in Table 2 below and were incubated at 37°C for 2 days and 8 days.

**Table 2**

| | Concentration of recombinant Klotho protein (µg/ml) | Incubation period (days) |
|---|---|---|
| Reference Example 1 | 0 | 2 |
| Reference Example 2 | 1 | 2 |
| Reference Example 3 | 0.5 | 2 |
| Reference Example4 | 0 | 8 |
| Reference Example 5 | 1 | 8 |
| Reference Example 6 | 0.5 | 8 |

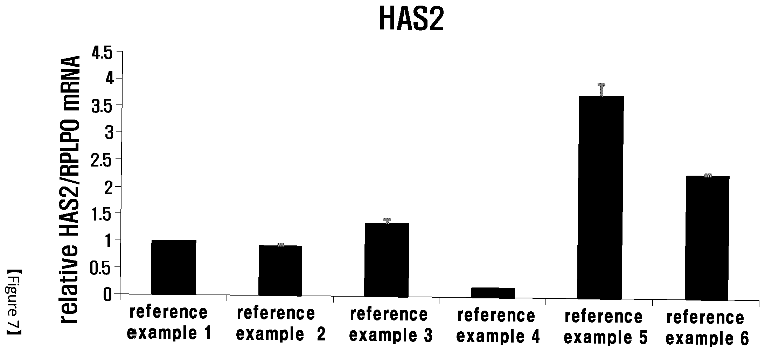

RNA was extracted from the cultured HEK cells using a RNeasy mini kit (Qiagen) and subjected to RT-PCR using a Superscript III kit (Invitrogen) to synthesize cDNA. The synthesized cDNA was subjected to quantitative real-time PCR using a probe (TaqMan® Gene Expression Assays) for HAS2, thereby analyzing the expression pattern of HAS2 mRNA. The results of the analysis are shown in FIG. 7.

As can be seen from the results in FIG. 7, the expression of HAS2 mRNA in reference example 4 (incubated for 8 days) was lower than at in reference example 1 (incubated for 2 days), and the expression of HAS2 mRNA in reference example 5 (incubated for 8 days) treated with the recombinant Klotho protein was significantly higher than that in reference example 1 (incubated for 2 days). The lower expression of HAS2 mRNA in reference example 4 than that in reference example 1 appears to be because of the differentiation and keratinization of HEK, and the expression of HAS2 in the cells treated with the recombinant Klotho protein was restored at 8 days, suggesting the Klotho protein functions to restore the moisturizing ability of the epidermal layer.

### Test Example 7: Increase in expression level of p21 in Klotho-knockdown human neonatal dermal fibroblasts (HDF)

In order to examine the influence of Klotho protein on p21 whose expression is known to increase with aging, the change in the expression level of p21 in Klotho-knockdown human neonatal dermal fibroblasts (HDF) was observed by RT-PCR.

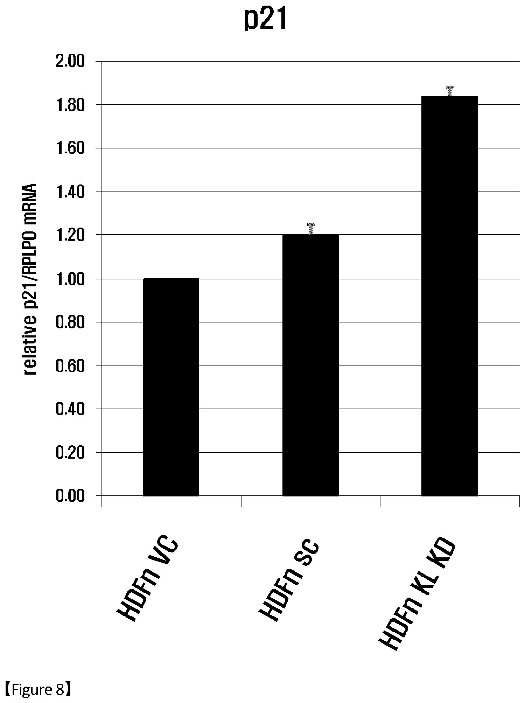

Human neonatal dermal keratinocytes (Lonza, NHEK-Neo-Neonatal Human Dermal Keratinocytes, Pooled) were dispensed in a 60-mm dish with DMEM medium at a density of 2x10⁵ cells and cultured at 37°C for 24 hours, after which the medium was replaced with OPTI-MEM medium (GIBCO). Meanwhile, human neonatal epidermal keratinocytes were cultured in OPTI-MEM medium (GIBCO) containing 100 nM of siRNA (small interfering RNA; Dharmacon, ON-TARGET*plus*) having a sequence complementary to Klotho gene and 10 µL of transfection reagent (RNAiMAX, Invitrogen) at 37°C for 20 minutes, after which these cells were added to the above cultured cells and incubated at 37°C for 6 hours. Then, the cells were incubated in DMEM medium at 37°C for 4 days. As control groups, HDF cells treated with siRNA (HEKn sc in FIG. 8) and HDF cells treated with only transfection reagent (RNAiMAX) without siRNA (HEKn VC in FIG. 8) were used.

RNA was extracted from the cultured HDF cells using a RNeasy mini kit (Qiagen) and subjected to RT-PCR using a Superscript III kit (Invitrogen) to synthesize cDNA. The synthesized cDNA was subjected to quantitative real-time PCR using a probe (TaqMan® Gene Expression Assays) for the senescence marker p21, thereby analyzing the expression pattern of p21 mRNA. The results of the analysis are shown in FIG. 8.

As can be seen from the results in FIG. 8, the expression level of p21 in the Klotho-knockdown cells was higher than that in the control group. This suggests that Klotho functions to inhibit the aging of skin dermal fibroblasts.

### Sequence List text

SEQ ID NOS: 1 and 2 are the DNA nucleotide sequences of Klotho protein amplification primers, and SEQ ID NO: 3 is the DNA nucleotide sequence of a TaqMan fluorogenic probe.

## Claims

1. Use of a kit for screening moisturizing substances by screening substances which increase the expression of Klotho gene in skin cells, wherein the kit comprises Klotho gene, Klotho gene amplification primers of SEQ ID NOS: 1 and 2, a probe of SEQ ID NO: 3, and skin cells.

2. The use of claim 1, wherein the skin cells are human keratinocytes, fibroblasts or melanocytes.

3. A method for screening moisturizing substances using the kit of claim 1 or 2, the method comprising the steps of
treating skin cells with candidates and determining whether Klotho gene is expressed in the skin cells treated with the candidates; and
isolating and purifying substances, which increase the expression of the Klotho gene, among the candidates.

## Patentansprüche

1. Verwendung eines Kits zum Screenen von Feuchthaltesubstanzen durch Screenen von Substanzen, welche die Expression von Klotho-Gen in Hautzellen erhöhen, wobei das Kit Klotho-Gen, Klotho-Gen-Amplifikationsprimer von SEQ ID NOS: 1 und 2, einer Sonde von SEQ ID NO: 3, und Hautzellen umfasst.

2. Verwendung nach Anspruch 1, wobei die Hautzellen Keratinozyten, Fibroblasten oder Melanozyten sind.

3. Verfahren zum Screenen von Feuchthaltesubstanzen unter Verwendung des Kits von Anspruch 1 oder 2, wobei das Verfahren die Schritte umfasst:
Behandeln der Hautzellen mit Kandidaten und Bestimmen, ob Klotho-Gen in den mit den Kandidaten behandelten Hautzellen exprimiert ist; und
Isolieren und Reinigen von Substanzen unter den Kandidaten, die die Expression von Klotho-Gen erhöhen.

## Revendications

1. Utilisation d'un kit de criblage de substances hydratantes en criblant les substances qui augmentent l'expression du gène Klotho dans des cellules de peau, lequel kit comprend le gène Klotho, des amorces d'amplification du gène Klotho de SEQ ID NO: 1 et 2, une sonde de SEQ ID NO: 3 et des cellules de peau.

2. Utilisation selon la revendication 1, dans laquelle les cellules de peau sont des kératinocytes, des fibroblastes ou des mélanocytes humains.

3. Procédé de criblage de substances hydratantes en utilisant le kit selon la revendication 1 ou 2, le procédé comprenant les étapes consistant à
traiter des cellules de peau avec les candidats et à déterminer si le gène Klotho est exprimé dans les cellules de peau traitées avec les candidats ; et
isoler et purifier les substances qui augmentent l'expression du gène Klotho parmi les candidats.


### Sequence Listing

<110> AMOREPACIFIC CORPORATION
<120> Detection kit for skin-a ctive ingredients comprising klotho gene and the method for detecting skin- active ingredients by using the same
<160> 3
<170> KopatentIn 1.71
<210> 1
   <211> 24
   <212> DNA
   <213> Artificial Sequence
<220>
   <223> Forward primer
<400> 1
   tggaaacctt aaaagccatc aagc 24
<210> 2
   <211> 20
   <212> DNA
   <213> Artificial Sequence
<220>
   <223> Reverse primer
<400> 2
   ccacgcctga tgctgtaacc 20
<210> 3
   <211> 28
   <212> DNA
   <213> Artificial Sequence
<220>
   <223> TaqMan fluorogenic probe
<400> 3
   ctgtgccact cgaaaccatc catgaggg 28